Europäisches Patentamt

European Patent Office

Office européen des brevets

(19)

(11) **EP 1 407 767 A1**

(12) **EUROPEAN PATENT APPLICATION**
published in accordance with Art. 158(3) EPC

(43) Date of publication:
**14.04.2004 Bulletin 2004/16**

(21) Application number: **02733509.0**

(22) Date of filing: **18.06.2002**

(51) Int Cl.[7]: **A61K 31/202**, A61P 3/06,
A61P 3/10, A61P 9/00,
A61P 9/10, A61P 29/00

(86) International application number:
**PCT/JP2002/006066**

(87) International publication number:
**WO 2002/102364 (27.12.2002 Gazette 2002/52)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **18.06.2001 JP 2001182731**

(71) Applicants:
• **Yamada, Sachico
  Hachioji-shi, Tokio 193-0845 (JP)**
• **Yamamoto, Keiko
  Tokyo 165-0032 (JP)**

(72) Inventors:
• **TAMAI, Tadakazu, c/o Maruha Corporation
  Tsukuba-shi, Ibaraki 300-4295 (JP)**
• **YOSHIKAI, Kazuyoshi, c/o Maruha Corporation
  Tsukuba-shi, Ibaraki 300-4295 (JP)**
• **NISHIKAWA, Masazumi, c/o Maruha Corporation
  Tsukuba-shi, Ibaraki 300-4295 (JP)**

(74) Representative: **HOFFMANN EITLE
Patent- und Rechtsanwälte
Arabellastrasse 4
81925 München (DE)**

(54) **PPARG AGONISTIC MEDICINAL COMPOSITIONS**

(57)     The present invention relates to PPARγ-activating pharmaceutical compositions comprising a hydroxylated derivative of a C20-22 polyunsaturated fatty acid or a pharmaceutically acceptable salt thereof, preferably PPARγ-activating pharmaceutical compositions containing a hydroxylated derivative of docosahexaenoic acid (DHA) or a hydroxylated derivative of eicosapentaenoic acid (EPA) or a pharmaceutically acceptable salt thereof, as well as uses of the PPARγ-activating pharmaceutical compositions for treating circulatory diseases, arteriosclerosis, lipid metabolism disorder, diabetes and inflammatory diseases.

EP 1 407 767 A1

**Description**

TECHNICAL FIELD

The present invention relates to pharmaceutical compositions containing a derivative of a polyunsaturated fatty acid. More specifically, the present invention provides such pharmaceutical compositions activating PPARγ (Peroxisome proliferator-activated receptor γ).

BACKGROUND ART

Peroxisome proliferator-activated receptors (PPARs) were first discovered as nuclear orphan receptors, and three types of genes for subtypes α, σ (or β) and γ were subsequently identified. Each of α, σ and γ has a specific tissue distribution and forms a subfamily of the nuclear hormone receptor gene superfamily. PPARγ is highly expressed in adipose tissue and seems to play an important role in adipocyte differentiation and fat production.

During searches for hypoglycemics, a report showed that thiazolidinedione (TZD) derivatives developed as drugs for improving insulin resistance might be ligands for PPARγ ('95 Cell. 83 pp. 803-812). The mechanism of blood sugar decrease by TZD still now remains unexplained about six years after then, but it was proposed that TZD might improve insulin resistance induced by impaired insulin signal transduction to reduce blood sugar by activating PPARγ in type 2 diabetes (NIDDM) ('99 Cell. 100 p. 1863). In brief, insulin secreted from pancreatic β cells binds to insulin receptors (IRs) in liver and peripheral skeletal muscle to promote glucose intake and utilization via activation of insulin receptor substrates (IRSs) in normals, but NIDDM patients show high blood glucose due to impaired transduction downstream of IRSs ('00 Science 289 p. 37, '00 J. Clin. Inv. 105, 10 p. 1437).

Knockout (KO) mice prepared by a research group of Kadowaki et al. by inactivating the IRS-1 gene among IRS subtypes showed insulin resistance but did not develop diabetes ('00 J. Clin. Inv. 105, 10 p. 1437). In NIDDM patients associated with the IRS-1-mediated signal transduction impaired (or IRS phosphorylation suppressed) by an inflammatory cytokine TNFα secreted from adipocytes and inflammatory cells, TZD recovered IRS-1 phosphorylation impaired by TNFα ('97 J. Clin. Inv. 100 p. 1863). Thus, it was shown that TZD recovered insulin signal transduction by activating IRS-1 to improve insulin resistance.

The research group of Kadowaki et al. attempted to prepare PPARγ-KO mice for the purpose of studying direct effects of PPARγ. As double homozygous KO mice (null) showed fetal death of lung failure, heterozygous KO mice were analyzed for convenience to show weight loss and suppression of adipocyte differentiation in heterozygous KO mice as compared with the wild-type having more PPARγ genes, suggesting that PPARγ may potentially act as a gene for storing energy in preparation against starvation ('99 Moll. Cell 4 p. 597). Thus, it is described that PPARγ stores energy by increasing the number of adipocytes to maintain homeostasis of carbohydrates and lipids.

Both insulin signal normalization and adipocyte differentiation by PPARγ activated by TZD seem to trigger TZD to reduce blood glucose, but TZD has the problem of producing strong side effects. Some TZD drugs commercialized for improving insulin resistance were withdrawn from the market due to severe hepatic toxicity or reported to invite death by hepatopathy. Therefore, PPARγ-mediated antidiabetic drugs free from side effects as produced by TZD would be desirable.

PPARγ agonists not only improve NIDDM but also improve high blood TG (triacylglycerol) or reduce cholesterol to improve lipid levels, indicating that they may potentially combat arteriosclerosis or improve lipid metabolism ('98 Diabetologia 41, p.257).

On the other hand, hydroxylated metabolites of arachidonic acid such as leukotriene or prostaglandin are known to induce pain, tissue damage and fever via neutrophil activation, platelet aggregation and vascular contraction. Hydroxylated derivatives of polyunsaturated fatty acids (PUFAs) found abundantly in fish oil such as docosahexaenoic acid (DHA, 22:6 n-3)(C22 linear unsaturated fatty acid of the n-3 series at a degree of unsaturation of 6) and eicosapentaenoic acid (EPA, 20:5 n-3) (C20 linear unsaturated fatty acid of the n-3 series at a degree of unsaturation of 5) were reported to be metabolized by lipoxygenase, which is an enzyme present in the skin and gills of red sea bream or rainbow trout. However, any cell type having lipoxygenase activity has not been identified. Last year, a research group of Andrew F. Rowley et al. in the UK fractionated gill cells of rainbow trout by density gradient using Percoll and studied lipid hydroxylation activity of the resulting cell populations to find that epithelial cells of gills show lipoxygenase activity ('99 Comparative Biochemistry and Physiology 122, p.297).

Rowley et al. adopted the following procedure. The gills of rainbow trout of 120-500 g body weight were minced in Hanks' buffer and digested with collagenase to give a cell suspension, which was then layered over Percoll and fractionated into 3 layers of cell populations by centrifugation at 20,000 x g for 15 minutes. The three cell populations were identified to be polymorphic epithelial cells, goblet epithelial cells and other cells by H & E staining and PAS staining and each cell population (1 x $10^7$ cells) was stimulated by $Ca^{2+}$ ionophores and then analyzed for fatty acids by reverse phase HPLC using a $C_{18}$ ODS column to show that the epithelial cell population produced OH-DHA and OH-EPA. This suggested that the previously reported lipoxygenase activity of the gills of fish may be localized at epithelial cells.

As to physiological effects of hydroxylated PUFAs found from fish epithelial cells, Norman Salem Jr.

et al. studied their effects on the contraction of vascular smooth muscle [J. W. Karanian et al., The Journal of Pharmacology and Experimental Therapeutics (1994) 270, 1105 - 1109.]. N. Salem Jr. et al. showed that four compounds, i.e. C11, C14 and C17 mono-OH-DHAs and C12 OH-EPA inhibit the contraction of rat aortic smooth muscle induced by a thromboxane $A_2$ (TX $A_2$) analog U 46619 (2.5 μM) with the potency being in the order of 14OH-DHA > 17OH-DHA > 11OH-DHA > 12OH-EPA, among which the most effective 140H-DHA showed a 25% inhibition concentration $IC_{25}$ of 1.07 ± 0.34 μM. This showed that hydroxylated PUFAs found from fish gills may potentially inhibit TX $A_2$-induced platelet aggregation or vascular contraction to have an antiinflammatory effect.

Also in mammals, lipoxygenase activity was reported in platelets and intestinal epithelial cells, e.g. 11OH-DHA and 14OH-DHA were shown to be metabolized from exogenous DHAs in human platelet. It was also shown that 1.4 μM 14OH-DHA was released by $Ca^{2+}$ ionophore stimulation from platelets of rats having received fish oil and that the $IC_{25}$ of the released product, i.e. the concentration at which contraction of vascular smooth muscle is inhibited to 25% was almost comparable to that of the 14OH-DHA described above. In other words, the concentration of 14OH-DHA in mammals agrees with the concentration range having antiinflammatory effect, suggesting that 14OH-DHA may positively contribute to maintaining the homeostasis in animal bodies.

However, no report has shown so far that such hydroxylated derivatives of polyunsaturated fatty acids act on PPARγ.

DISCLOSURE OF THE INVENTION

An object of the present invention is to develop a novel PPARγ-activating pharmaceutical composition and to provide it as a drug for treating various diseases in which PPARγ is involved.

As a result of careful studies to attain the above object, we accomplished the present invention on the basis of the finding that hydroxylated derivatives of C20-22 polyunsaturated fatty acids show PPARγ agonist activity.

Accordingly, the present invention provides a PPARγ-activating pharmaceutical composition comprising a hydroxylated derivative of a C20-22 polyunsaturated fatty acid or a pharmaceutically acceptable salt thereof.

As used herein, the PPARγ-activating pharmaceutical composition means a pharmaceutical product for preventing/treating various diseases in which PPARγ is involved by acting on PPARγ, which is a nuclear receptor in cells such as adipocytes, to activate it.

The present invention also provides said PPARγ-activating pharmaceutical composition for treating circulatory diseases.

The present invention also provides said PPARγ-activating pharmaceutical composition for treating arteriosclerosis.

The present invention also provides said PPARγ-activating pharmaceutical composition for treating lipid metabolism disorder.

The present invention also provides said PPARγ-activating pharmaceutical composition for treating diabetes.

The present invention also provides said PPARγ-activating pharmaceutical composition for treating inflammatory diseases.

BRIEF EXPLANATION OF THE DRAWINGS

FIG. 1 is a graph showing the results of a PPARγ agonist activity assay of various OH-DHA compounds.
FIG. 2 is a graph showing the results of an evaluation of whether or not collagen-induced ex vivo platelet aggregation is inhibited by 4(S)-OH-DHA.
FIG. 3 is a graph showing the results of an evaluation of whether or not 4(S)-OH-DHA dose-dependently inhibits ex vivo platelet aggregation.
FIG. 4 is a graph showing the influence of 4(S)-OH-DHA on the interaction between neutrophils and vascular endothelial cells (neutrophil migration (%)).
FIG. 5 is a graph showing the influence of 4(S)-OH-DHA on the interaction between neutrophils and vascular endothelial cells (neutrophil adhesion (%)).

THE MOST PREFERRED EMBODIMENTS OF THE INVENTION

PPARγ-activating pharmaceutical compositions of the present invention contain a hydroxylated derivative of a C20-22 polyunsaturated fatty acid as an active ingredient.

As used herein, the C20-22 polyunsaturated fatty acid means a polyunsaturated fatty acid containing 20-22 carbon atoms and three or more double bonds, preferably 4 or more, more preferably 5 or 6 double bonds. Preferred polyunsaturated fatty acids include, but not limited to, docosahexaenoic acid (DHA, 22:6 n-3)(C22 linear unsaturated fatty acid of the n-3 series at a degree of unsaturation of 6) and eicosapentaenoic acid (EPA, 20:5 n-3) (C20 linear unsaturated fatty acid of the n-3 series at a degree of unsaturation of 5).

The hydroxylated derivative of a polyunsaturated fatty acid means a derivative of the polyunsaturated fatty acid in which any one of double bonds is hydroxylated, preferably a hydroxylated derivative of docosahexaenoic acid (DHA) or a hydroxylated derivative of eicosapentaenoic acid (EPA), more preferably a hydroxylated derivative of docosahexaenoic acid (DHA). The hydroxylated derivative may be in either the (R) or (S) configuration, preferably the (S) configuration. The most preferred hydroxylated derivatives of docosahexaenoic acid (DHA) are, but not limited to, 4(S)-OH-DHA, 10(S)-OH-DHA, 11(S)-OH-DHA, 14(S)-OH-DHA, 8(S)-OH-DHA and 17(S)-OH-DHA. PPARγ-activating pharmaceutical compositions of the present invention can contain one or more members selected from these hydroxylated derivatives of polyunsaturated fatty acid.

The hydroxylated derivatives of polyunsaturated fatty acids can be prepared by any process, e.g. they can be isolated/purified from gills or epithelial cells of fish or platelets of mammals such as human and rat. They can also be synthesized by hydroxylating DHA from a natural source and fractionating the hydroxylated DHA by HPLC or the like. For example, a suspension of gill cells or epithelial cells of rainbow trout, mammalian platelets or a humanized leukocyte cell line such as RBL-1 can be reacted with 10-200 mM DHA as a substrate at 10-37°C for 1-50 minutes. The reaction is stopped by acidifying the reaction solution (e.g. with formic acid, acetic acid or trichloroacetic acid), and each OH derivative is extracted with an organic solvent (e.g. chloroform, methanol, ethyl acetate or acetonitrile) and then fractionated by a method such as, but not limited to, HPLC or thin layer chromatography using a developing solvent (e.g. chloroform, methanol, ethyl acetate, acetonitrile, water or trifluoroacetic acid). Each OH derivative can also be prepared by a selective synthetic process using a site-specific enzyme. 4(S)-OH-DHA, 10(S)-OH-DHA, 11(S)-OH-DHA, 14(S)-OH-DHA, 8(S)-OH-DHA and 17(S)-OH-DHA are commercially available from Wako Pure Chemical Industries, Ltd.

PPARγ-activating pharmaceutical compositions of the present invention are effective for preventing/treating various diseases in which PPARγ is involved by acting on PPARγ, which is a nuclear receptor in cells such as adipocytes, to activate it. The diseases in which PPARγ is involved include circulatory diseases (e.g. thrombosis, myocardial infarction pectoris, angina, cerebral infarction), arteriosclerosis, lipid metabolism disorder (e.g. hyperlipidemia, hypercholesterolemia, high TG, high LDL), diabetes (especially NIDDM), and inflammatory diseases (e.g. various inflammatory responses caused by increased vascular permeability).

PPARγ-activating pharmaceutical compositions of the present invention are normally administered systemically or locally, orally or parenterally. The dose is not specifically limited but should be optimally determined on the basis of overall judgment depending on various factors such as the type of the disease, the severity of the condition, the age and body weight of the subject to be treated. However, the daily dose is normally 0.001 to 100 mg/kg orally or 0.0001 to 10 mg/kg parenterally per adult. The dose is administered once daily or divided into subdoses depending on the purpose.

The hydroxylated derivatives of polyunsaturated fatty acids of the present invention as active ingredients may be pharmaceutically acceptable salts thereof including acid addition salts such as methanesulfonates, fumarates, hydrochlorides, citrates, maleates, tartrates and hydrobromides.

Pharmaceutical compositions of the present invention may be administered orally in the form of solid compositions, liquid compositions and other compositions or parenterally in the form of injections, external preparations and suppositories, and an optimal administration mode is selected depending on the purpose. The pharmaceutical compositions can be prepared by using carriers, excipients and other additives used for ordinary formulation. Suitable carriers and excipients for formulation include, for example, lactose, magnesium stearate, starch, talc, gelatin, agar, pectin, gum acacia, olive oil, sesame oil, cacao butter, ethylene glycol and other common additives.

Suitable solid compositions for oral administration include tablets, pills, capsules, powders and granules. In such solid compositions, at least one active substance (active ingredient) is mixed with at least one inert diluent such as lactose, mannitol, glucose, hydroxypropylcellulose, microcrystalline cellulose, starch, polyvinyl pyrrolidone or magnesium aluminometasilicate. The compositions may conventionally contain additives other than inert diluents, e.g. lubricants such as magnesium stearate; disintegrants such as calcium cellulose glycolate; and solubilizers such as glutamic acid or aspartic acid. Tablets or pills may be coated with a sugar coating such as sucrose, gelatin or hydroxypropyl methylcellulose phthalate, or a film of a gastric soluble or enteric material, or two or more layers. Capsules of absorbable materials such as gelatin are also included.

Liquid compositions for oral administration include pharmaceutically acceptable emulsions, solutions, suspensions, syrups and elixirs, and may contain ordinary inert diluents such as purified water and ethanol. In addition to inert diluents, these compositions may contain adjuvants such as wetting agents or suspending agents, sweetening agents, flavoring agents, aromatics and preservatives.

Injections for parenteral administration include sterile aqueous or nonaqueous solutions, suspensions and emulsions. Aqueous solutions and suspensions contain e.g. water for injection and physiological saline for injection. Nonaqueous solutions and suspensions include e.g. propylene glycol, polyethylene glycol, vegetable oils such as olive oil, alcohols such as ethanol, and Polysorbate® 80. These compositions may further contain adjuvants such as preservatives, wetting agents, emulsifying agents, dispersing agents, stabilizers (e.g. lactose), and solubilizers (e.g. glutamic acid and aspartic acid). These can be sterilized by ordinary sterilizing methods, such as mechanical sterilization with a microfiltration membrane, heat sterilization such as autoclaving or inclusion of a bactericide. A solid composition can also be prepared and dissolved in sterile water or a sterile solvent for injection before use.

Other pharmaceutical compositions for parenteral administration include liquid preparations for external use, ointments, liniments, suppositories, transdermal preparations and ophthalmic solutions containing at least one of compounds of the present invention as an active ingredient.

The active ingredients can be prepared as O/W emulsions using emulsifiers such as phospholipids or nonionic surfactants as described in JPA HEI6-298642. The emulsifiers can be used alone or in combination at an appropriate concentration, e.g. 0.001-10% (W/V), preferably 0.01-5%(W/V).

Suitable phospholipids include soy phospholipids, egg yolk phospholipids, lysolecithin, phosphatidylcholine (lecithin) and phosphatidylserine, which can be used alone or in combination. Preferred nonionic surfactants include, but not limited to, polyoxyethylene-polyoxypropylene block copolymers of molecular weight 500-15000 (e.g. Pluronic F-68), polyalkylene glycols of molecular weight 1000-10000, polyoxyalkylene copolymers of molecular weight 1000-20000, hydrogenated castor oil polyoxyalkylene derivatives, castor oil polyoxyalkylene derivatives, glycerin fatty acid esters, polyglycerin fatty acid esters, sorbitan fatty acid esters, polyoxyethylene castor oils, hydrogenated castor oils, polyoxyethylene alkyl ethers and sucrose fatty acid esters, which can be preferably used alone or in combination.

As shown in the examples below, hydroxylated derivatives of docosahexaenoic acid (DHA) were assayed for PPARγ agonist activity to show remarkable PPARγ agonist activity. That is, all of 8(S)-OH-DHA, 14(S)-OH-DHA, 4(S)-OH-DHA, 10(S)-OH-DHA, 11(S)-OH-DHA and 17(S)-OH-DHA activated PPARγ at an activity comparable to or higher than that of 15-deoxy-PG J2 used as a positive control.

Moreover, hydroxylated derivatives of docosahexaenoic acid (DHA) significantly decreased blood glucose levels when they were orally administered to hereditary diabetes db/db mice.

In an ex vivo platelet aggregation assay, a hydroxylated derivative of docosahexaenoic acid (DHA) dose-dependently inhibited platelet aggregation.

In an assay using an in vitro model simulating vascular inflammation, the hydroxylated derivative of docosahexaenoic acid (DHA) was shown to inhibit both adhesion and permeation of neutrophils. This suggests that the hydroxylated derivative of docosahexaenoic acid (DHA) may potentially act on the interaction between neutrophils and vascular endothelial cells in a suppressive manner to have an antiinflammatory effect and can be expected to maintain homeostasis of the circulatory system and act to improve pathologies by their antiinflammatory effect.

The hydroxylated derivatives of polyunsaturated fatty acids forming active ingredients of PPARγ-activating pharmaceutical compositions of the present invention are derived from natural sources so that they can be expected to have little side effects such as toxicity. Thus, PPARγ-activating pharmaceutical compositions of the present invention are very useful as antidiabetic drugs, especially for preventing and/or treating type 2 diabetes (NIDDM) without side effects as produced by TZD. PPARγ-activating pharmaceutical compositions of the present invention can also be expected to be effective as drugs for treating inflammatory diseases.

The following examples further illustrate the present invention without, however, limiting the scope of the invention thereto. Various changes and modifications can be made by those skilled in the art on the basis of the description of the invention, and such changes and modifications are also included in the present invention.

## EXAMPLES

Example 1: Preparation of hydroxylated derivatives of DHA

Gills of rainbow trout (body weight: 3 g) were minced in 0.05 M PBS and suspended for 30 seconds using a Polytron homogenizer. An enzyme solution was obtained by centrifugation at 150,000 x g for 15 minutes. This enzyme solution (10 $\mu$l) was reacted in 0.9 ml of 0.05 M PBS (1 mM reduced glutathione, 25°C, pH 7.8). Into the reaction solution was added 70 mM DHA for oxidation reaction for 10 minutes, and the reaction was stopped by adjusting the solution to pH 4 with 3% formic acid. The reaction solution was extracted with ethyl acetate, and lipids were fractionated by HPLC using chloroform : methanol : acetic acid : water (90 : 8 : 1 : 0.8) as a developing solvent. The products were analyzed by GC-MS to confirm that they were 4(S)-OH-DHA, IO(S)-OH-DHA, 11(S)-OH-DHA, 14(S)-OH-DHA (m/z 430 [M], 373 [M-CH$_3$]$^+$, 340 [M-HOSi(CH$_3$)$_3$]$^+$), 8(S)-OH-DHA, and 17(S)-OH-DHA. These compounds can also be available from Wako Pure Chemical Industries, Ltd.

Example 2: PPARγ agonist activity assay

COS-1 cells were cotransfected with a plasmid for expressing a GAL4 (yeast transcription activator) - PPARγ fusion protein (effector plasmid) and a reporter plasmid 17 M$_2$ CAT (GAL4 responsive sequence + TK promoter + chloramphenicol acetyltransferase cDNA), and then test compounds were added to the culture medium and CAT activity was assayed after 24 hours and compared with a negative control (with no drug added) to calculate a relative CAT activity, whereby PPARγ agonist activity was evaluated ('90 Proc. Natl. Acad. Sci. USA 87, p. 9995; '94 J. B. C. 269, p. 32700; '95 ibid. p. 5858; '00 J.B.C. 275, p. 33201).

Experiments using DHA (from Cayman Chemical) and EPA (from Cayman Chemical) as test compounds each at 3 μM showed that DHA significantly increased PPARγ transcription activity at 3 μM in a ratio of 205±79% as compared with the negative control while no activity was observed with 3 μM EPA.

Then, 4(S)-OH-DHA, 8(S)-OH-DHA, 10(S)-OH-DHA, 11(S)-OH-DHA, 14(S)-OH-DHA and 17(S)-OH-DHA prepared in Example 1 were used as test compounds to assay PPARγ agonist activity as compared with 15-deoxy-PG J2 (from Bio Mol) known as a ligand for PPARγ as a positive control.

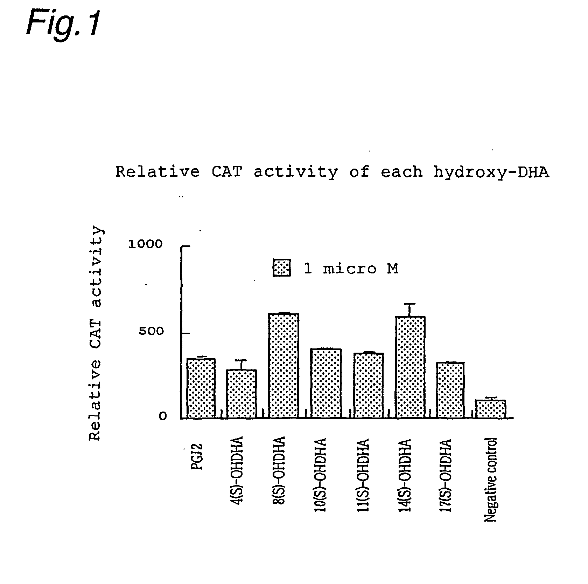

The results are shown in FIG. 1. 8(S)-OH-DHA and 14(S)-OH-DHA each at a concentration of 1 μM showed about 7 times higher activity than that of the negative control group with no drug added, and 4(S)-OH-DHA, 10(S)-OH-DHA, 11(S)-OH-DHA, and 17(S)-OH-DHA at the same concentration showed about 3 times higher activity than that of the negative control, i. e. all the compounds had an agonist activity comparable to or higher than that of 15-deoxy-PG J2 used as a positive control

Example 3: Evaluation on hereditary diabetes mice

As a result of oral administration of 100 mg/kg of 8(S)-OH-DHA and 14(S)-OH-DHA to hereditary diabetes db/db mice (average body weight: 42 g, n=6, available from Sankyo Co., Ltd.) for a week, a significant decrease in blood glucose levels could be observed as compared with a negative control group (treated with 5% gum acacia as a vehicle) (Table 1).

Table 1

| Compound | Blood glucose level (mg/dl) |
|---|---|
| Negative control | 320 ± 40 |
| 8(S)-OH-DHA | 190 ± 20 * |
| 14(S)-OH-DHA | 220 ± 13 * |

*: p<0.05 vs. negative control.

Example 4: Ex vivo platelet aggregation assay

An evaluation was made to determine whether or not collagen-induced platelet aggregation is inhibited by 4(S)-OH-DHA.

(1) Preparation of platelets

Male SD rats (SPF, 9 weeks of age) were anesthetized and then blood was collected from the abdominal aorta into a tube containing 3.8% sodium citrate (Citral from Yamanouchi Pharmaceutical Co., Ltd.). The tube was centrifuged at 900 rpm at room temperature for 10 minutes to collect the supernatant (PRP, platelet-rich fraction), and the residue was further centrifuged at 2500 rpm for 15 minutes to collect the supernatant (PPP, platelet-poor fraction).

(2) Determination of transmittance

A cuvette containing PPP (platelet-poor fraction) was placed in an incubator for PPP at 37°C in an aggregometer (Hematracer 240 12ch, from MCM). Then, a cuvette containing 200 μl of PRP (platelet-rich fraction) was placed in a reactor for PRP. The cuvettes were stirred with a magnetic stirrer for 30 seconds to automatically correct the transmittance of PPP to T650nm=100% and the transmittance of PRP to T650 nm=0%.

After 1 minute, 20 μl of collagen (from MCM) was added and subsequent changes in transmittance were recorded for 5 minutes. The transmittance increases with platelet aggregation. The transmittance T at maximum aggregation, i.e. at the peak of aggregation was compared with the transmittance T0 of a negative control to determine the degree of aggregation inhibition expressed in percentage using the following equation.

$$\text{Aggregation inhibition} = (1\text{-T/T0}) \times 100$$

(3) Evaluation of the onset of pharmaceutical efficacy

The aggregation induced by 3-6 μg/ml of collagen was evaluated in platelets prepared from blood collected at 2 instants, i.e. 10 minutes and 60 minutes after administration of 10 mg/kg of 4(S)-OH-DHA to the tail vain of male SD rats (SPF, 9 weeks of age). The aggregation was determined in animals treated with vehicle (physiological saline) as a control group.

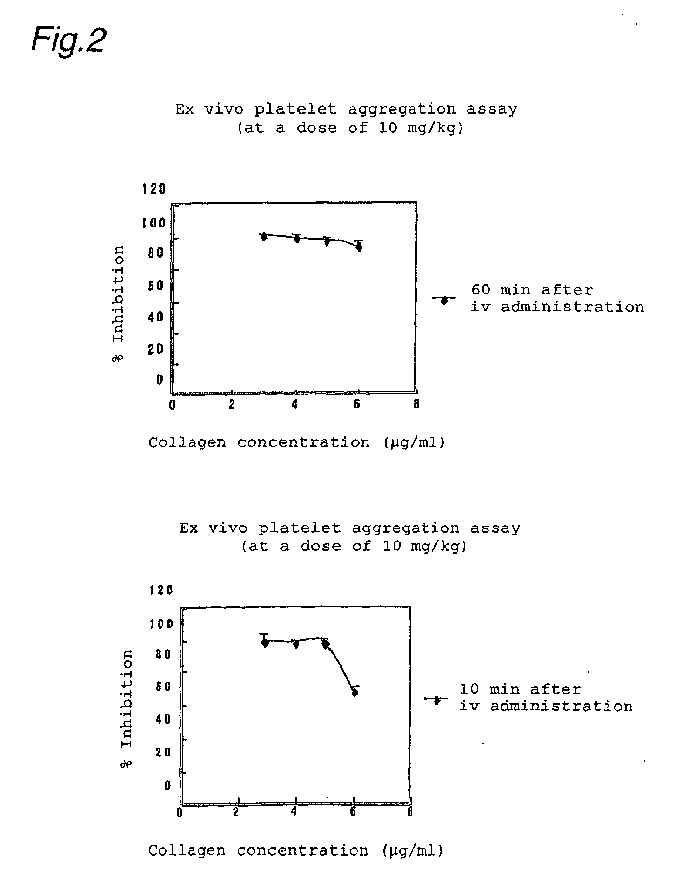

The results are shown in FIG. 2.

At both 10 minutes and 60 minutes after administration of 4(S)-OH-DHA, platelet aggregation was inhibited as compared with the vehicle group and the efficacy was almost comparable at both instants.

(4)Evaluation of dose-dependency

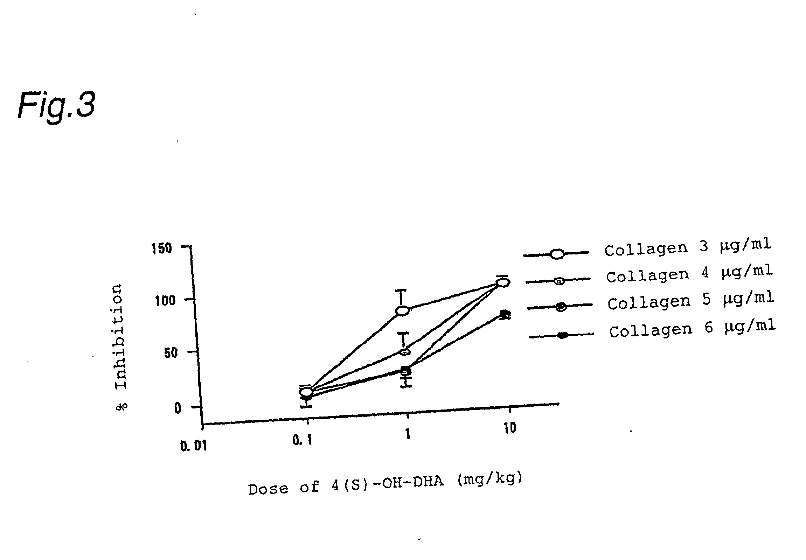

An evaluation was made to determine whether or not 4(S)-OH-DHA dose-dependently inhibits ex vivo platelet aggregation. As a control, a solvent (physiological saline) was used. Aggregation was induced by 3-6 μg/ml of collagen in platelets prepared from blood collected 10 minutes after administration of 10, 1 or 0.1 mg/kg of the test compound to the tail vain of rats. As a result, platelet aggregation was inhibited in the groups treated with 10 and 1 mg/kg as compared with the vehicle group (FIG. 3).

Example 5: Evaluation on an in vitro model simulating vascular inflammation

Reports show that when an inflammatory cytokine TNFα induces adhesion molecules on vascular endothelial cells to cause inflammation, neutrophils migrate to inflamed sites to further promote inflammation.

Severe inflammation is thought to fail homeostasis of the circulatory system to advance arteriosclerosis.

PPARγ agonists were reported to reduce adhesion of inflamed cells to vascular endothelial cells to act to inhibit inflammation by suppressing the expression of a TNFα-induced adhesion molecule ICAM-1 in vascular endothelial cells ('00 Circulation 101, p.235).

Thus, an evaluation was made to determine whether or not 4(S)-OH-DHA having PPARγ agonist activity has an antiinflammatory effect in an in vitro model simulating vascular inflammation induced by TNFα.

(1) Establishment of an in vitro model simulating vascular inflammation

An in vitro model simulating vascular inflammation was prepared according to the method described in "Textbook on Biopharmaceutical Experiments, 12 Inflammation and Allergy II", pp. 327-341 (edited by Kazuo Ohuchi, published by Hirokawa Publishing Co., May 15, 1993).

Transwells (from Kurabo Industries, Ltd.) divided into upper and lower chambers by a porous polycarbonate membrane having a pore size of 3 μm were used and a layer of bovine endothelial cells were coated on the bottom of the membrane and incubated at 37°C under 5% $CO_2$ for 80 minutes. Then, a suspension of fluorescent-labeled neutrophils was added to the upper chamber of each transwell and at the same time TNFα was suspended into the cell suspension in the upper chamber at a final concentration of 50, 25 or 17 ng/ml.

Thus, the above model in which neutrophils permeate a layer of vascular endothelial cells from the upper chamber into the lower chamber and adhere to the layer of endothelial cells simulates the migration of neutrophils to inflamed sites from the inside of the vessel in response to TNFα in the vessel.

(2) Influence of 4(S)-OH-DHA on the interaction between neutrophils and vascular endothelial cells

Then, the above in vitro model simulating vascular inflammation was used to evaluate how 4(S)-OH-DHA influences the interaction between neutrophils and vascular endothelial cells.

To the upper chamber of the transwells was added a suspension of neutrophils containing 4(S)-OH-DHA at a final concentration of 30, 3 or 0.3 μM together with TNFα to count the number of neutrophils permeating the vascular endothelial cell layer from the upper chamber into the lower chamber and the number of neutrophils adhering to the endothelial cell layer in the same manner as described above, and thereafter the migration and adhesion (%) of neutrophils were calculated.

The migration and adhesion percentages of neutrophils were expressed as relative values of the numbers of neutrophils that migrated or adhered in the group treated with the drug as compared with neutrophils in a negative control group.

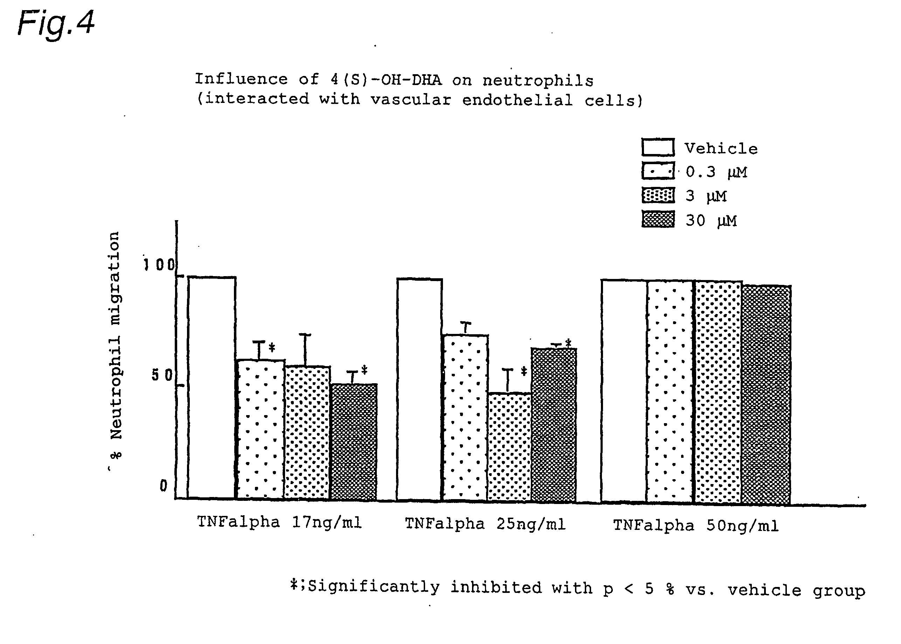

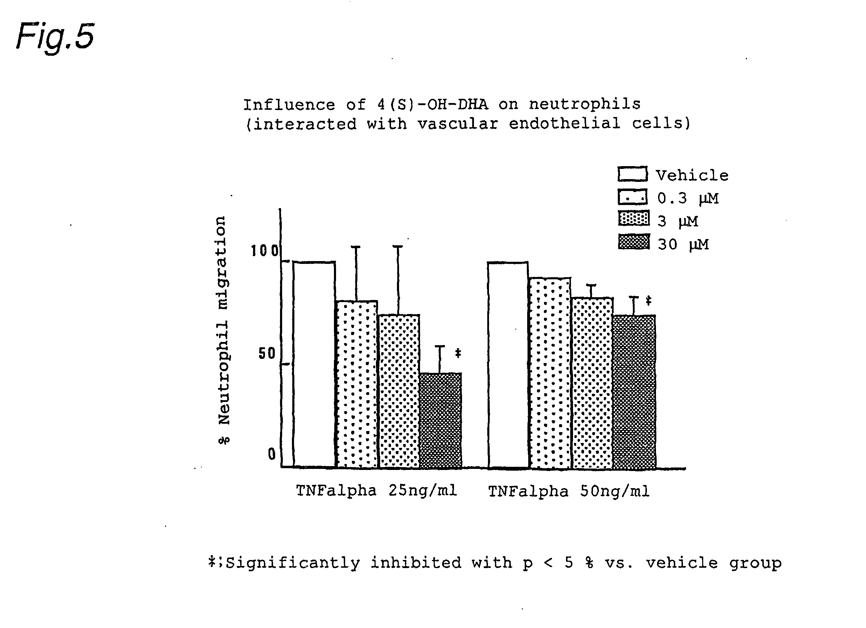

The results are shown in FIG. 4 and FIG. 5. 4 (S)-OH-DHA inhibited both adhesion and permeation of neutrophils. Thus, it was suggested that 4(S)-OH-DHA could act on the interaction between neutrophils and vascular endothelial cells in a suppressive manner to have an antiinflammatory effect.

4(S)-OH-DHA may potentially maintain homeostasis of the circulatory system and act to improve pathologies by its antiinflammatory effect.

**Claims**

1. A PPARγ-activating pharmaceutical composition comprising a hydroxylated derivative of a C20-22 polyunsaturated fatty acid or a pharmaceutically acceptable salt thereof.

2. The PPARγ-activating pharmaceutical composition of claim 1 wherein the hydroxylated derivative of a polyunsaturated fatty acid is a hydroxylated derivative of docosahexaenoic acid (DHA) or a hydroxylated derivative of eicosapentaenoic acid (EPA).

3. The PPARγ-activating pharmaceutical composition of claim 2 wherein the hydroxylated derivative of docosahexaenoic acid (DHA) is at least one member selected from 4(S)-OH-DHA, 10(S)-OH-DHA, 11(S)-OH-DHA, 14(S)-OH-DHA, 8(S)-OH-DHA and 17(S)-OH-DHA.

4. The PPARγ-activating pharmaceutical composition of any one of claims 1 to 3 for treating circulatory diseases.

5. The PPARγ-activating pharmaceutical composition of any one of claims 1 to 3 for treating arteriosclerosis.

6. The PPARγ-activating pharmaceutical composition of any one of claims 1 to 3 for treating lipid metabolism disorder.

7. The PPARγ-activating pharmaceutical composition of any one of claims 1 to 3 for treating diabetes.

8. The PPARγ-activating pharmaceutical composition of any one of claims 1 to 3 for treating inflammatory diseases.

*Fig.1*

Relative CAT activity of each hydroxy-DHA

## Fig.2

Ex vivo platelet aggregation assay
(at a dose of 10 mg/kg)

Collagen concentration (μg/ml)

Ex vivo platelet aggregation assay
(at a dose of 10 mg/kg)

Collagen concentration (μg/ml)

Fig.3

Fig.4

Influence of 4(S)-OH-DHA on neutrophils
(interacted with vascular endothelial cells)

*;Significantly inhibited with p < 5 % vs. vehicle group

## Fig.5

EP 1 407 767 A1

Influence of 4(S)-OH-DHA on neutrophils
(interacted with vascular endothelial cells)

*;Significantly inhibited with p < 5 % vs. vehicle group

## INTERNATIONAL SEARCH REPORT

International application No.

PCT/JP02/06066

### A. CLASSIFICATION OF SUBJECT MATTER
Int.Cl$^7$  A61K31/202, A61P3/06, 3/10, 9/00, 9/10, 29/00

According to International Patent Classification (IPC) or to both national classification and IPC

### B. FIELDS SEARCHED
Minimum documentation searched (classification system followed by classification symbols)
Int.Cl$^7$  A61K31/202, A61P3/06, 3/10, 9/00, 9/10, 29/00

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 5-186342 A  (Fujirebio Inc.),<br>27 July, 1993 (27.07.93),<br>Particularly, Claims<br>(Family: none) | 8 |
| A | JP 2000-355538 A  (Kaneka Corp.),<br>26 December, 2000 (26.12.00),<br>& WO 00/62772 A1       & EP 1175901 A1 | 1-8 |
| A | JP 63-17825 A  (Teijin Ltd.),<br>25 January, 1988 (25.01.88),<br>(Family: none) | 1-8 |
| A | WO 96/34943 A1  (City of Hope),<br>07 November, 1996 (07.11.96),<br>& JP 11-511004 A       & EP 824583 A1<br>& US 6191169 B1 | 1-8 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 July, 2002 (22.07.02) | 06 August, 2002 (06.08.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

| International application No. |
|---|
| PCT/JP02/06066 |

| C (Continuation). DOCUMENTS CONSIDERED TO BE RELEVANT | | |
|---|---|---|
| Category* | Citation of document, with indication, where appropriate, of the relevant passages | Relevant to claim No. |
| P,A | JP 2001-354558 A (Kao Corp.), 25 December, 2001 (25.12.01), (Family: none) | 1-8 |
| E,A | JP 2002-186424 A (Kaneka Corp.), 02 July, 2002 (02.07.02), (Family: none) | 1-8 |

Form PCT/ISA/210 (continuation of second sheet) (July 1998)